# EUROPÄISCHE PATENTSCHRIFT

(11) **EP 2 664 288 B1**
(45) Veröffentlichungstag und Bekanntmachung des Hinweises auf die Patenterteilung: **09.03.2016**
(21) Anmeldenummer: 13002567.9
(22) Anmeldetag: 16.05.2013
(51) Int. Cl.: A61B 17/32

(54) **Motorhandstück**
Motor driven hand piece
Pièce à main motorisée

(30) Priorität: 18.05.2012 DE 102012010579
(43) Veröffentlichungstag der Anmeldung: 20.11.2013
(73) Patentinhaber: Ladwig Feinwerktechnik GmbH, 75181 Pforzheim (DE)
(72) Erfinder: Ladwig, Andreas, 75181 Pforzheim (DE)
(74) Vertreter: Wacker, Jost Oliver

(56) Entgegenhaltungen:
- GB-A- 1 519 513
- US-A- 6 132 448
- US-A1- 2002 038 102
- US-A1- 2002 040 229

## Beschreibung

Die Erfindung betrifft ein Motorhandstück eines Bearbeitungsgerätes für die medizinische oder kosmetische Fußpflege, die Chirurgie oder für den Dentalbereich nach dem Oberbegriff des Anspruchs 1. Dieses Motorhandstück weist ein Gehäuse auf, in dem zwischen einem Anschlussende und einem Bearbeitungsende ein Aufnahmeraum vorgesehen ist, in dem ein elektrischer Motor sowie Lager, wie insbesondere Kugellager, zum Antrieb eines am Bearbeitungsende anbringbaren Werkzeuges aufgenommen werden können. Ferner ist ein Kühlpfad zur Führung eines fluidförmigen Kühlmediums vorgesehen, der benachbart zum Aufnahmeraum angeordnet ist. Das Kühlmedium kann dabei beispielsweise aus einer Flüssigkeit, wie insbesondere Wasser, und/oder Luft bestehen.

Der benachbart zum Aufnahmeraum angeordnete Kühlpfad ermöglicht hierbei die Ableitung von Wärme, die insbesondere bei längerer Betriebsdauer des Motors erzeugt wird. Dies schützt einerseits den Motor und alle anderen Bauteile des Motorhandstückes vor einer schädlichen Überhitzung. Ferner wird auf diese Weise gewährleistet, dass das Motorhandstück an seiner Oberfläche auch bei längerer Betriebsdauer eine Temperatur aufweist, die dem Benutzer eine komfortable Handhabung ermöglicht.

Aus WO 02/24083 A2 und US 2002/0040229 A1 ist ein chirurgisches Handstück gemäß dem Oberbegriff des Anspruchs 1 bekannt, dessen Motor in einem fluiddichten, zylindrischen Gehäuse untergebracht ist. Über diesem Gehäuse ist eine zylindrische Gehäusehülse vorgesehen, deren Innendurchmesser.größer ist als der Außendurchmesser des Motorgehäuses. Über den hierdurch gebildeten Zwischenraum wird dabei ein fluidförmiges Kühlmedium von einem Einlass zu einem Auslass geführt, mittels dem die vom Motor im Betrieb erzeugte Wärme abgeleitet werden kann.

Nachteilig an dem bekannten Motorhandstück ist, dass die Abdichtung des Kühlpfades sowohl nach Außen als auch zum Motor hin relativ aufwändig ist und es nach längerer Betriebszeit dennoch häufig zu Undichtigkeiten kommt. Ein weiterer Nachteil besteht darin, dass bei dieser Bauweise die Verlegung weiterer Funktionsleitungen entlang des Motors nur an der Außenseite der Gehäusehülse möglich ist. Zudem weist der Kühlpfad des bekannten Motorhandstückes mehrere beträchtliche Querschnittssprünge auf, so dass es in bestimmten Bereichen zu einem zu geringen Kühleffekt in Folge von unzureichendem Wasseraustausch kommen kann, der beispielsweise aus Kurzschlussströmungen in aufgeweiteten Abschnitten resultieren kann.

US 6,132,448 beschreibt ein endoskopisches Schneidewerkzeug mit einem werkzeugseitigen Ende, an dem eine Flüssigkeit ausgesprüht werden kann. Um dabei die Flüssigkeit entlang des Schneidewerkzeuges transportieren zu können, ist in eine äußere Gehäusehülse eine Längsnut eingelassen.

US 2002/0038102 A1 zeigt ein System für den Betrieb verschiedener Mikro-Schneideinstrumente. Dabei ist eine separate Bevorratung von Kühlmittel und Sprühmedium vorgesehen, wobei eine Drehzahl des Motors abhängig von einem Füllstand eines Vorratsspeichers gesteuert ist.

DE 31 36 889 A1 zeigt einen Kleinmotor für den Antrieb medizinischer Instrumente. Um dessen Kühlung zu ermöglichen, wird auf ein zylindrisches Gehäuse des Motors eine ebenfalls kreiszylindrische Hülse aufgeschoben, die zusammen mit dem Motorgehäuse einen Durchflusskanal für ein Kühlmittel bildet.

DE 10 2007 025 009 A1 beschreibt einen Stator einer elektrischen Werkzeugmaschine, bei der unterschiedlich genutzte Freiräume dadurch gebildet werden, dass sich ein Motorgehäuse über radiale Innenrippen an einer Feldwicklung des Motors abstützt.

DE 101 22 425 B4 zeigt eine elektrische Maschine mit einem wassergekühlten Motorgehäuse. Hierzu weist das Motorgehäuse einen zweiteiligen Kühlmantel mit einer Außenhülse auf, an deren Innenseite halbrunde Kühlkanäle eingelassen sind, die an einer Innenhülse anliegen.

DE 2431472 C2 und GB 1,519,513 beschreiben ein zahnärztliches Handstück, bei dem Kühlleitungen vorgesehen sind, die im Bereich des Motorgehäuses durch hierin verlegte Leitungskanäle gebildet sind. Außerhalb des Motorgehäuses sind die Kühlleitungen dagegen teilweise durch Bohrungen in einem ansonsten massiven Dreh- oder Spritzteil gebildet.

DE 28 52 763 A1 zeigt ein zahntechnisches Werkzeug, bei dem ein Strömungsringraum zwischen einem zylindrischen Gehäuse und einer Antriebswelle gebildet wird.

DE 197 14 466 A1 beschreibt einen Elektromotor, bei dem an einer Innenseite eines zylindrischen Gehäuses Fluidleitungen schraubenförmig verlegt sind, die wiederum zusammen mit dem Gehäuse schraubenförmige Zwischenräume bilden.

Die Aufgabe der Erfindung ist es, bei einem gattungsgemäßen Motorhandstück die genannten Nachteile zu vermeiden und eine einfache und dauerhaft störungsfreie Kühlfunktion zu gewährleisten.

Diese Aufgabe wird durch das Motorhandstück mit den Merkmalen des Anspruchs 1 gelöst. Dabei ist der Kühlpfad durch wenigstens eine Materialausnehmung im Material der Gehäusewand gebildet, das heißt in Form eines Hohlraumes in die Gehäusewand des Gehäuses integriert und gegenüber dem Motor abgedichtet. Auf diese Weise wird der Kühlpfad zusammen mit dem übrigen Gehäuse einstückig zur Verfügung gestellt und gehandhabt, was eine leichtere Montage des Motorhandstückes und eine geringere Anzahl von Einzelteilen gewährleistet. Ferner kann der Fluidpfad auf diese Weise gegenüber dem Motor oder anderen Funktionselementen des Motorhandstücks problemlos abgedichtet werden.

Dabei ist es günstig, wenn der Kühlpfad um den Aufnahmeraum herum mehrere zueinander beabstandete Kühlabschnitte aufweist, die beispielsweise über Krümmungsabschnitte miteinander verbunden sind, um auch über lediglich einen beispielsweise mäandernden Kühlkreislauf, der sich über eine große Fläche an der Außenseite des Motors und den Lagern erstreckt eine möglichst gute Wärmeabführung sicherstellen zu können.

Vorteilhafterweise sind die Kühlabschnitte durch im Wesentlichen parallele, spiralförmige, oder unregelmäßig ausgebildete Hohlräume gebildet, von denen jeweils zwei benachbarte Hohlräume an einem Ende miteinander verbunden sind. Hier durch kann die bei der Wärmeabführung wirksame Austauschfläche, die den Aufnahmeraum beziehungsweise den Motor umgibt, besonders groß beziehungsweise weitestgehend geschlossen ausgeführt werden, was wiederum eine bessere Kühlwirkung gewährleistet.

Zudem ist es günstig, wenn in der Gehäusewand ein Fluid führender Funktionspfad vorgesehen ist, der sich vom Anschlussende zum Bearbeitungsende hin erstreckt und über den ein am Bearbeitungsende angeordnetes Funktionselement mit einem Fluid beaufschlagbar ist, das beispielsweise aus einer Flüssigkeit, wie insbesondere Wasser, und/oder Luft sowie gegebenenfalls weiterer Zusätze besteht. Auf diese Weise kann die Gehäusewand neben der Kühlung des Motors oder eines Lagers beispielsweise auch zur Versorgung einer Sprühdüse genutzt werden, wobei auch über den Fluid führenden Funktionspfad eine gewisse Kühlwirkung erzielt werden kann.

Vorteilhafterweise ist der Fluid führende Funktionspfad dabei im Gehäuse separat zum Kühlpfad in die Gehäusewand integriert. Durch diese parallele Ausführung des Kühlpfades zu dem wenigstens einen Funktionspfad kann der Durchfluss beziehungsweise der Druck des den Kühlpfad durchströmenden Kühlmediums unabhängig vom Durchfluss beziehungsweise des Druckes des wenigstens einen Fluid führenden Funktionspfades gewählt werden. Zudem kann im Gegensatz zu einem gegenüber dem Fluid führenden Funktionspfad in Reihe angeordneter Kühlpfad ein zum Funktionsfluid des Funktionspfades unterschiedliches Kühlmedium verwendet werden.

In einer weiteren vorteilhaften Ausführungsform ist zusätzlich zu dem Fluid führenden Funktionspfad wenigstens eine weitere Funktionsaussparung in die Gehäusewand integriert. Auf diese Weise können neben der Führung von Fluiden weitere Funktionselemente in der Gehäusewand untergebracht werden, die beispielsweise zur Aufnahme von Kabeln und/oder Schläuchen dienen oder Ausnehmungen zur Gewichtsreduzierung bilden. Hierdurch lassen sich die für den Betrieb des Motorhandstückes wesentlichen Funktionselemente in die Gehäusewand integrieren, wodurch das Motorhandstück auch bei mehreren benötigten Leitungen mit einem relativ kleinen Durchmesser hergestellt werden kann. Zudem kann auf diese Weise eine dauerhaft sichere Trennung von beispielsweise Flüssigkeit führenden Leitungen gegenüber Luft führenden Leitungen oder Strom führenden Leitungen gewährleistet werden.

Ferner ist es günstig, wenn die Gehäusewand am Anschlussende mit einem Verteilerelement verbunden ist, in das Verteilerleitungen zur Verbindung des Kühlpfades und dem wenigstens einen Funktionspfad mit einer jeweils passenden Versorgungsleitung eingelassen sind. Ein solches Verteilerelement ermöglicht bei der Montage des Motorhandstückes eine besonders einfache Verbindung der in das Motorhandstück integrierten Leitungen mit einer jeweils passenden Versorgungsleitung, die beispielsweise neben weiterer Versorgungsleitungen in einem Versorgungsleitungstrang angeordnet ist.

Dabei ist es in jedem Fall günstig, wenn wenigstens die Gehäusewand und vorteilhafterweise zusätzlich auch das Verteilerelement im Laser-Sinter-Verfahren hergestellt ist. Hierdurch kann sowohl die Gehäusewand als auch gegebenenfalls das Verteilerelement mit einer relativ komplexen Formgebung problemlos hergestellt werden. Hierbei kann insbesondere die Form und der Verlauf der Höhlräume des Kühlpfades relativ frei gewählt werden, um eine möglichst optimale Wärmeabführung gewährleisten zu können. Auch der Verlauf der Verteilerleitungen kann hierbei im Falle, dass auch das Verteilerelement im Laser-Sinter-Verfahren hergestellt ist, relativ frei gewählt werden, um beispielsweise eine leichte Verbindung mit den Versorgungsleitungen herstellen zu können. Zudem kann bei dieser Herstellungsart der Gehäusewand, beispielsweise im Falle von Motorhandstücken aus dem Dentalbereich, auch die Durchleitung von Sprühfluiden in die Gehäusewand verlegt werden, wodurch auf die übliche Verlegung von separaten Röhrchen verzichtet werden kann.

In einer weiteren vorteilhaften Ausführungsform ist dabei wenigstens die Gehäusewand aus einer Metallegierung wie einer Edelstahl-, Aluminium- oder Titanlegierung oder aus Kunststoff, wie beispielsweise Polyamid, hergestellt, wodurch einerseits auch durch das Material der Gehäusewand hindurch eine gute Wärmeleitung und zudem eine gewisse Langlebigkeit des Motorhandstückes und der Lager gewährleistet werden kann.

Ferner ist es günstig, wenn Ober den Fluid führenden Funktionspfad eine am Bearbeitungsende angeordnete Sprühdüse mit einer Sprühflüssigkeit beaufschlagbar ist, wodurch neben der Kühlung des Motors im Motorhandgriff auch in dessen Arbeitsbereich eine Kühlwirkung erzielt werden kann.

In einer besonders vorteilhaften Ausführungsform ist sowohl die Sprühflüssigkeit als auch das Kühlmedium durch Wasser gebildet, das in einem gemeinsamen Vorratsspeicher bevorratet ist. Auf diese Weise kann das Bearbeitungsgerät für die Motorkühl- und Sprühfunktion mit einem einzigen Vorratsbehälter versehen werden, wodurch dieser insgesamt einfacher und kompakter ausgeführt werden kann.

Alternativ hierzu ist es günstig, wenn das Sprühfluid und das Kühlmedium in zwei getrennten Vorratsspeichern bevorratet sind. Auf diese Weise kann das Kühlmedium in einem geschlossenen Kreislauf zwischen dem Vorratsspeicher und dem Motorhandstück zirkulieren, ohne dass ein wesentlicher Verbrauch desselben erfolgt. Somit kann auch über eine lange Betriebsdauer hinweg jederzeit eine ausreichende Kühlung des Motorhandstückes gewährleistet werden. Der getrennte Vorratsspeicher des Sprühfluids kann dagegen bedarfsweise nachgefüllt werden oder kann aus einem stationären Versorgungsnetz bestehen, wie beispielsweise einer Trinkwasserversargung, um auch eine dauerhafte Versorgung des Funktionselementes sicherstellen zu können.

Vorteilhafterweise ist ferner eine Drehzahl des Motors in Abhängigkeit eines Füllstandes des Vorratsspeichers für das Kühlmedium steuerbar, um bereits vor einer vollständigen Leerung des Vorratsspeichers den Kühlbedarf zu reduzieren, um dadurch einen weiteren Betrieb des Motofiandstückes zu ermöglichen, ohne dass es zu Schäden kommt, und um dem Benutzer anzuzeigen, dass das Kühlmedium nachgefüllt werden muss.

Ferner ist es alternativ oder zusätzlich hierzu günstig, wenn das Sprühfluid und/oder das Kühlmedium mittels eines Kompressors erzeugbar ist, wodurch das jeweilige Fluid beziehungsweise Luft/Wasser-Gemisch mit einem ausreichenden Druck zur Verfügung gestellt werden kann.

Darüber hinaus ist es ferner vorteilhaft wenn eine Kühleinrichtung vorgesehen ist, mittels der das Kühlmedium gekühlt werden kann, wobei die Kühlung bevorzugterweise in einer Rücklaufleitung zwischen dem Motorhandstück und dem Vorratsbehälter erfolgt Auf diese Weise weist das im Vorratsbehählter zur Verfügung stehende Kühlmedium auch nach längerer Betriebszeit des Bearbeitungsgerätes eine relativ niedrige Temperatur auf, um eine ausreichende Kühlung des Motorhandstückes gewährleisten zu können.

In den Figuren ist eine beispielhafte Ausführungsform der Erfindung dargestellt. Es zeigen:
- Figur 1: ein Bearbeitungsgerät für die medizinische oder kosmetische Fußpflege,
- Figur 2: eine perspektivische Ansicht eines Motorhandstückes des Bearbeitungsgerätes,
- Figur 3: eine explodierte Ansicht des Motorhandstückes nach Fig. 2 und
- Figur 4: eine Draufsicht auf ein Verteilerelement des Motorhandstückes in Richtung IV aus Fig. 3.

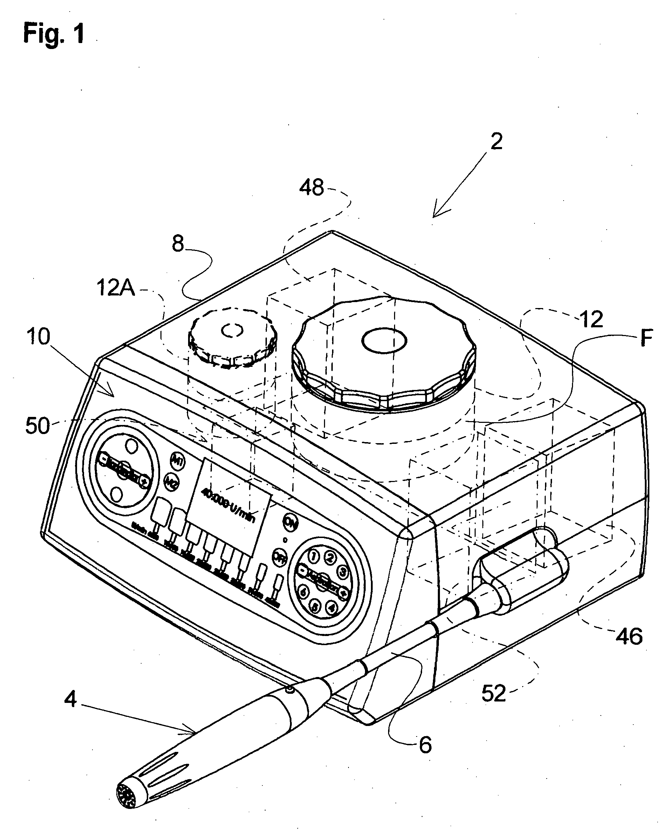

Fig. 1 zeigt ein Bearbeitungsgerät 2 in Form eines Gerätes zur medizinischen oder kosmetischen Fußpflege, das ein Motorhandstück 4 aufweist. Das Motorhandstück 4 ist dabei über eine Versorgungsleitung 6 mit einem Basisgerät 8 verbunden, das einen Steuerungsteil 10 und einen Vorratsspeicher 12 für eine Flüssigkeit F aufweist, die sowohl als Kühlmedium wie auch als Sprühfluid für das Motorhandstück 4 dient, und beispielsweise durch Wasser, gegebenenfalls unter Verwendung von Zusätzen, gebildet ist.

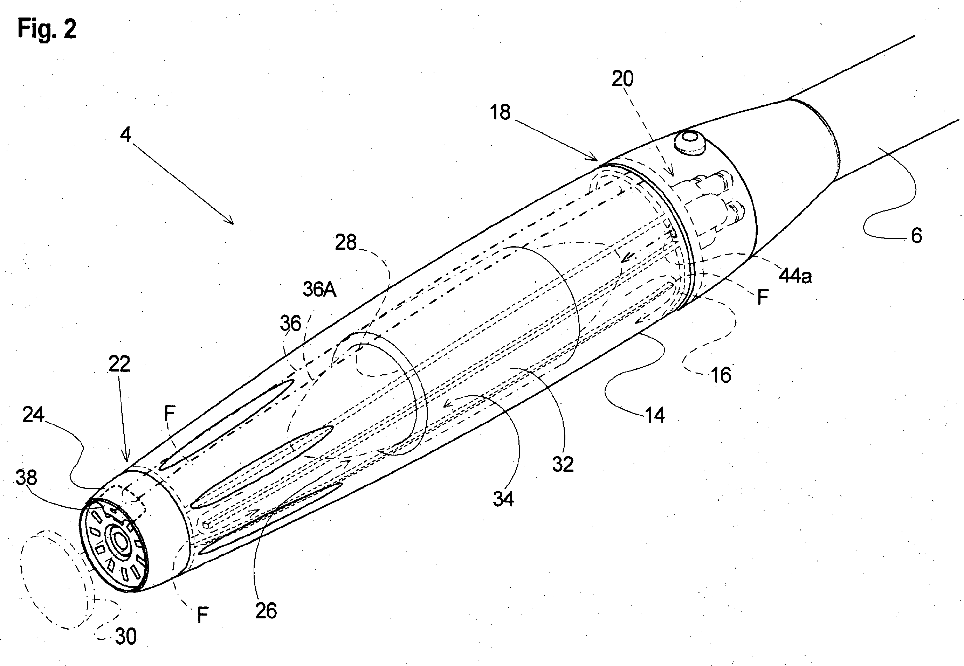

Wie insbesondere aus Fig. 2 zu entnehmen ist, weist das Motorhandstück 4 ein Gehäuse 14 mit einer Gehäusewand 16 auf, die aus Edelstahl, Aluminium, einer Titan- oder sonstigen Metallegierung oder aus Kunststoff besteht und in einem Laser-Sinter-Verfahren hergestellt ist. Dieses Gehäuse 14 weist zwischen einem Anschlussende 18, an dem ein Verteilerelement 20 vorgesehen ist und einem Bearbeitungsende 22, an dem ein Endstück 24 angebracht ist, einen Aufnahmeraum 26 auf. Dieser Aufnahmeraum 26 dient dabei zur Aufnahme eines elektrischen Motors 28, mittels dem ein an dem Bearbeitungsende 22 anbringbares Werkzeug 30 angetrieben werden kann.

Die Gehäusewand 16 ist derart in einem Laser-Sinter-Verfahren aufgebaut, dass sie um den Aufnahmeraum 26 herum mehrere Ausnehmungen 32 aufweist, die einen Kühlpfad 34 bilden, entlang dessen die als Kühlmedium dienende Flüssigkeit F während des Betriebs des Berabeitungsgerätes 2 geführt werden kann.

In dem in Fig. 2 dargestellten Ausführungsbeispiel sind die Ausnehmungen 32 durch im Wesentlichen parallele Hohlräume gebildet, von denen jeweils zwei benachbarte Hohlräume immer abwechselnd am Anschlussende 18 und am Bearbeitungsende 22 über gekrümmte Abschnitte miteinander verbunden sind, so dass der Kühlpfad 34 um den Aufnahmeraum 26 herum mäandert und sich dabei ober einen großen Teil von dessen Außenseite erstreckt. Alternativ zu der im Wesentlichen parallelen Anordnung können die Hohlräume jedoch auch spiralförmig oder unregelmäßig um den Aufnahmeraum 26 herum angeordnet sein und sich dabei gegebenenfalls auch vollständig, das heißt über 360° um den Aufnahmeraum 26 herum erstrecken.

Separat zu dem Kühlpfad 34 ist in die Gehäusewand 16 in einem Bereich, über den sich der Kühlpfad 34 nicht erstreckt, zudem ein Fluid führender Funktionspfad 36 in Form einer Sprühleitung eingelassen. Über diese Sprühleitung kann das Sprühfluid, das beispielsweise aus dem Vorratsspeicher 12 entnommen wird, einem Funktionselement 38 in Form einer Sprühdüse zugeführt werden, die am Bearbeitungsende 22 angeordnet ist Auf diese Weise kann ein Sprühnebel erzeugt werden, der im Betrieb des Motorhandstückes 4 in dessen Arbeitsbereich beispielsweise für eine Kühlwirkung sorgt oder Staubpartikel benetzt, um eine unnötige Ausbreitung derselben zu verhindern (nicht dargestellt). Zudem wird über den Funktionspfad auch in dem zum Aufnahmeraum 26 benachbarten Bereich der Gehäusewand, Ober den sich der Kühlpfad 34 nicht erstreckt, eine gewisse Kühlwirkung durch das hierin transportierte Sprühfluid erzielt.

Bei der dargestellten Ausführungsform des Motorhandstückes 4 ist dabei ein weiterer Fluid führender Funktionspfad 36A vorgesehen, über den dem Funktionselement 38 beispielsweise Luft zugeführt werden kann, um mit dieser einen besonders feinen Sprühnebel erzeugen zu können. Darüber hinaus ist es auch möglich, in der Gehäusewand 16 weitere Funktionsaussparungen vorzusehen, die beispielsweise zur Gewichtsreduzierung oder zur Aufnahme von Kabeln oder Schläuchen dienen können (nicht dargestellt).

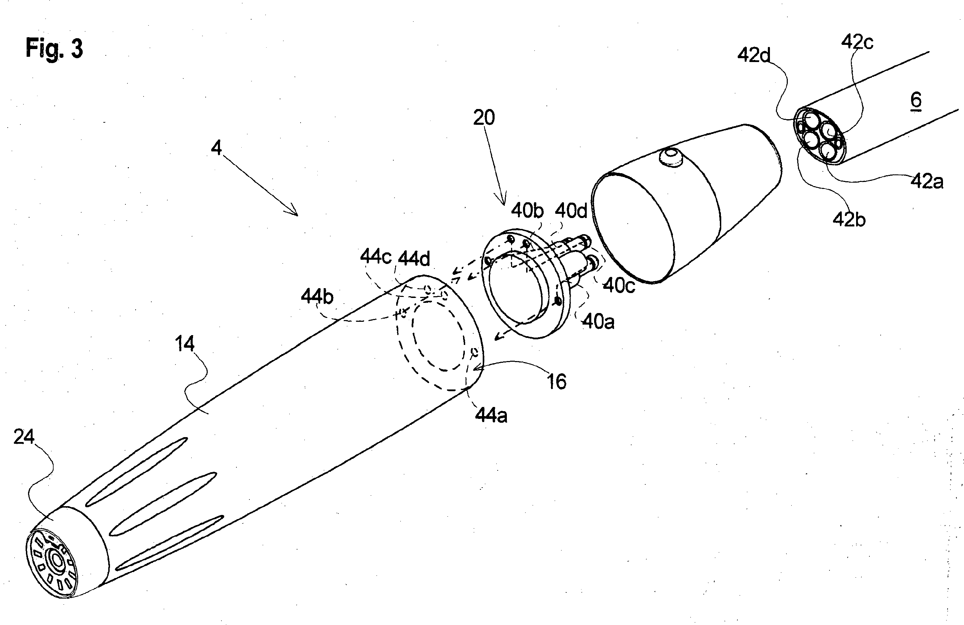

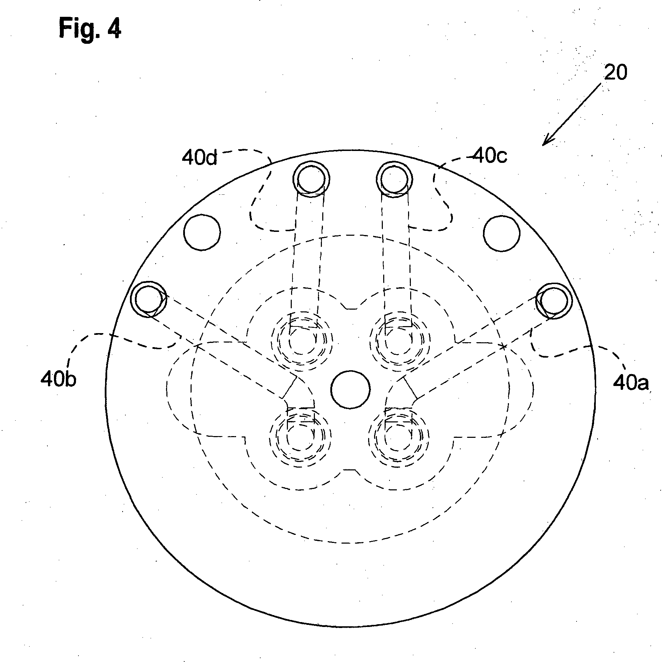

Um bei der Montage des Motorhandstückes 4 den in die Gehäusewand 16 eingelassenen Kühlpfad 34 und die Funktionspfade 36, 36A besonders leicht mit der Versorgungsleitung 6 verbinden zu können, sind in das Verteilerelement 20 Verteilerleitungen 40a, 40b, 40c, 40d eingelassen, wie aus Fig. 3 und 4 zu entnehmen ist. Diese Verteilerleitungen 40a, 40b, 40c, 40d verbinden dabei jeweils eine Versorgungsteilleitung 42a, 42b, 42c, 42d mit einer stirnseitigen Öffnung 44a, 44b, 44c, 44d der Gehäusewand 16, die mit dem jeweils passenden Funktionspfad 36, 36A beziehungsweise Kühlpfad 34 verbunden ist. Die Versorgungsleitung 6 weist hierbei für das Kühlfluid eine Zulaufleitung zum Motorhandstück 4 in Form der Versorgungsteilleitung 42a und eine Rücklaufleitung zurück zum Vorratsspeicher 12 in Form der Versorgungsteilleitung 42b auf. Ferner dienen die Versorgungsteilleitung 42c, die Verteilerleitung 40c und die Öffnung 44c zusammen mit dem Funktionspfad 36A zum Transport der Flüssigkeit F und die Versorgungsteilleitung 42d, die Verteilerleitung 40d und die Öffnung 44d zusammen mit dem Funktionspfad 36B zum Transport von Luft zum Funktionselement 38.

Auf diese Weise kann im Betrieb des Bearbeitungsgerätes 2 die Flüssigkeit F aus dem Vorratsspeicher 12 über die Versorgungsleitung 6 und die Verteilerleitungen 40 in die Gehäusewand 16 eingeleitet werden, um dort zum Wärmeabtransport über den Kühlpfad 34 oder zur Beaufschlagung des Funktionselementes 38 zu dienen.

Das Verteilerelement 20 mit den darin eingelassenen Verteilerleitungen 40a, 40b, 40c, 40d besteht dabei aus Edelstahl, einer Titanlegierung oder aus Kunststoff und ist wie auch die Gehäusewand 16 in einem Laser-Sinter-Verfahren hergestellt. In gleicher Weise und aus dem gleichen Material kann auch das Endstück 24 mit dem Funktionselement 38 hergestellt sein.

In dem gezeigten Ausführungsbeispiel ist die dem Kühlpfad 34 als Kühlmedium zugeführte Flüssigkeit F durch Wasser gebildet. Dabei ist eine Pumpeneinrichtung 46 vorgesehen, mittels der die Flüssigkeit F aus dem Vorratsspeicher 12 zum Motorhandstück 4 und wieder zurück befördert werden kann.

Ferner dient die Pumpeneinrichtung 46 über den fluidführenden Funktionspfad 36 auch zur Beaufschlagung des Funktionselementes 38 mit dem Wasser aus dem Vorratsspeicher 12, wobei über den weiteren Funktionspfad 36A gleichzeitig Druckluft von einem Kompressor 48 zum Funktionselement 38 befördert wird, um an diesem den im Betrieb benötigten Sprühnebel erzeugen zu können.

Alternativ zu dem sowohl für den Kühlpfad 34 als auch für den Funktionspfad 36 genutzten Vorratsspeicher 12 kann, wie in Fig. 1 durch strichpunktierte Linien angedeutet neben dem Vorratsspeicher 12 ein weiterer Vorratsspeicher 12A vorgesehen werden, um den Kühlpfad 34 gegenüber dem Funktionspfad 36 separat zu versorgen. Alternativ oder zusätzlich zu dem eigenen Vorratsspeicher 12 für die Versorgung des Funktionspfades 36 kann zudem ein Anschluss an ein Versorgungsnetz (nicht dargestellt), wie insbesondere an eine Wasserleitung vorgesehen werden, um auch die Sprühfunktion über eine längere Betriebsdauer aufrecht erhalten zu können.

Bei Versorgung des Funktionselementes 38 aus einem der Vorratsspeicher 12; 12A kann im Steuerungsteil 10 zudem eine Drehzahlsteuerung 50 vorgesehen sein, die bei Unterschreitung eines vorbestimmten Füllstandes des betreffenden Vorratsbehälters 12, 12A eine Drehzahl des Motors 32 verringert, um dessen Kühlbedarf zu reduzieren. Dabei kann der niedrige Füllstand dem Benutzer neben der Drehzahlreduzierung auch durch optische oder akustische Signale angezeigt werden.

In jedem Fall wird für das Kühlmedium ein geschlossener Kreislauf zwischen dem Vorratsspeicher 12; 12A und dem Motorhandstück 4 vorgesehen, bei dem zusätzlich in der als Rücklaufleitung dienenden Versorgungsteilleitung 42b eine Kühleinrichtung 52 vorgesehen werden kann, mittels der die Temperatur der Flüssigkeit F beim Zurückfließen zum Vorratsbehälter 12, 12A verringert werden kann.

Vorzugsweise sind zudem sowohl die Pumpeneinrichtung 46 als auch der Kompressor 48 in das Bearbeitungsgerät 2 integriert, um dieses in einer besonders vielfältigen und leicht zu handhabenden Ausführungsform zur Verfügung zu stellen. Alternativ hierzu können die Pumpeneinrichtung 46 und/oder der Kompressor 48 oder der Vorratsspeicher 12; 12A auch als separate Einheiten vorgesehen werden, um beispielsweise das Gewicht oder die Größe des Bearbeitungsgerätes 2 minimieren zu können.

In jedem Fall gewährleistet das Bearbeitungsgerät 2 mit dem fluidgekühlten Motorhandstück 4 auch bei längerer Betriebszeit des Motors 28 eine begrenzte Außentemperatur des Gehäuses 14 und dadurch ein angenehmes Halten des Motorhandstückes 4.

## Patentansprüche

1. Motorhandstück (4) eines Bearbeitungsgerätes (2) für die medizinische oder kosmetische Fußpflege, die Chirurgie oder für den Dentalbereich
mit einem Gehäuse (14), in dem zwischen einem Anschlussende (18) und einem Bearbeitungsende (22) ein Aufnahmeraum (26) vorgesehen ist, in dem ein elektrischer Motor (28) zum Antrieb eines am Bearbeitungsende (22) anbringbaren Werkzeuges (30) aufnehmbar ist,
und mit einem Kühlpfad (34) zur Führung eines Kühlmediums, der benachbart zum Aufnahmeraum (26) angeordnet ist,
**dadurch gekennzeichnet, dass** der Kühlpfad (34) durch Ausbildung wenigstens einer Materialausnehmung in Form eines gegenüber dem Motor (28) abgedichteten Hohlraumes in einer einstückigen Gehäusewand (16) in das Gehäuse (14) integriert ist.

2. Motorhandstück nach Anspruch 1, **dadurch gekennzeichnet, dass** der Kühlpfad (34) um den Aufnahmeraum (26) herum mehrere Kühlabschnitte aufweist.

3. Motorhandstück nach Anspruch 2, **dadurch gekennzeichnet, dass** die Kühlabschnitte durch im Wesentlichen parallele, spiralförmige, oder unregelmäßig ausgebildete Hohlräume gebildet sind, von denen jeweils zwei benachbarte Hohlräume an einem Ende miteinander verbunden sind.

4. Motorhandstück nach einem der Ansprüche 1 bis 3, **dadurch gekennzeichnet, dass** in der Gehäusewand (16) ferner ein Fluid führender Funktionspfad (36; 36A) vorgesehen ist, der sich vom Anschlussende (18) zum Bearbeitungsende (22) hin erstreckt und über den ein am Bearbeitungseride (22) angeordnetes Funktionselement (38) mit einem Fluid beaufschlagbar ist.

5. Motorhandstück nach Anspruch 4, **dadurch gekennzeichnet, dass** der Fluid führende Funktionspfad (36; 36A) separat zum Kühlpfad (34) in die Gehäusewand (16) integriert ist.

6. Motorhandstück nach Anspruch 5, **dadurch gekennzeichnet, dass** zusätzlich zu dem Fluid führenden Funktionspfad (36; 36A) wenigstens eine weitere Funktionsaussparung (zur Gewichtsreduzierung oder Aufnahme für Kabel oder Schläuche) in die Gehäusewand (16) integriert ist.

7. Motorhandstück nach einem der Ansprüche 1 bis 6, **dadurch gekennzeichnet, dass** die Gehäusewand (16) am Anschlussende (18) mit einem Verteilerelement (20) verbunden ist, in das Verteilerleitungen (40a, 40b, 40c, 40d) zur Verbindung des Kühlpfades (34) und des wenigstens einen Funktionspfades (36; 36A) mit einer jeweiligen Versorgungsteilleitung (42a, 42b, 42c, 42d) eingelassen sind.

8. Motorhandstück nach einem der Ansprüche 1 bis 7, **dadurch gekennzeichnet, dass** die Gehäusewand (16) im Laser-Sinter-Verfahren hergestellt ist.

9. Motorhandstück nach Anspruch 8, **dadurch gekennzeichnet dass** zusätzlich auch das Verteilerelement (20) im Laser-Sinter-Verfahren hergestellt ist.

10. Motorhandstück nach Anspruch 8 oder 9, **dadurch gekennzeichnet, dass** wenigstens die Gehäusewand (16) aus Metall oder Kunststoff hergestellt Ist.

11. Motorhandstück nach einem der Ansprüche 1 bis 10, **dadurch gekennzeichnet, dass** über die Fluid führende Funktionsleitung (36; 36A) eine am Bearbeitungsende (22) angeordnete Sprühdüse mit einem Sprühfluid beaufschiagbar ist.

12. Motorhandstück nach Anspruch 11, **dadurch gekennzeichnet, dass** sowohl das Sprühfluid als auch das Kühlmedium durch Wasser und/oder Luft mit oder ohne Zusätze gebildet und in einem gemeinsamen Vorratsspeicher (12) bevorratet.

13. Motorhandstück nach Anspruch 11, **dadurch gekennzeichnet, dass** das Sprühfluid und das Kühlmedium in zwei getrennten Vorratsspeichern (12, 12A) bevorratet sind.

14. Motorhandstück nach Anspruch 12 oder 13, **dadurch gekennzeichnet, dass** eine Drehzahl des Motors (28) in Abhängigkeit eines Füllstandes des Vorratsspeichers (12; 12A) für das Kühlmedium steuerbar ist.

15. Motorhandstück nach einem der Ansprüche 11 bis 14, **dadurch gekennzeichnet, dass** das Sprühfluid und/oder das Kühlmedium durch einen Kompressor(48) erzeugbar ist.

16. Motorhandstück nach einem der Ansprüche 1 bis 15, **dadurch gekennzeichnet, dass** eine Kühleinrichtung (52) vorgesehen ist, mittels der das Kühlmedium bevorzugterweise in einer Rücklaufleitung kühlbar ist.

## Claims

1. Motor-driven hand piece (4) of a processing device (2) for medical or cosmetic foot care, surgery, or for the dental sector,
with a housing (14), in which an accommodation space (26) is provided between a connection end (18) and a processing end (22), in which an electric motor (28) can be accommodated for the drive of a tool (30) which can be attached to the processing end (22),
and with a cooling path (34) for conducting a cooling medium, arranged adjacent to the accommodation space (26),
**characterized in that** the cooling path (34) is integrated into the housing (14) by the formation of at least one material cut-out opening in the form of a cavity, sealed against the motor (28), in a single-piece housing wall (16).

2. Motor-driven hand piece according to claim 1, **characterized in that** the cooling path (34) comprises a plurality of cooling sections around the accommodation space (26).

3. Motor-driven hand piece according to claim 2, **characterized in that** the cooling sections are formed by essentially parallel, helical, or irregularly formed cavities, of which in each case two adjacent cavities are connected to each other at one end.

4. Motor-driven hand piece according to any one of claims 1 to 3, **characterized in that**, additionally provided in the housing wall (16) is a function path (36; 36A) conducting fluid and extending from the connection end (18) to the processing end (22) and by which fluid can be supplied to a function element being (38) arranged at the processing end (22).

5. Motor-driven hand piece according to claim 4, **characterized in that** the function path (36; 36A) conducting fluid is integrated into the housing wall (16) separately from the cooling path (34).

6. Motor-driven hand piece according to claim 5, **characterized in that**, in addition to the function path (36; 36A) conducting the fluid, at least one further function cut-out opening (for reducing weight or to accommodate cables or hoses) is integrated into the housing wall (16).

7. Motor-driven hand piece according to any one of claims 1 to 6, **characterized in that** the housing wall (16) is connected at the connection end (18) to a distributor element (20), into which are let distribution lines (40a, 40b, 40c, 40d) for the connection of the cooling path (34) and of the at least one function path (36; 36A) with a respective supply distribution line (42a, 42b, 42c, 42d).

8. Motor-driven hand piece according to any one of claims 1 to 7, **characterized in that** the housing wall (16) is produced by the laser-sinter process.

9. Motor-driven hand piece according to claim 4, **characterized in that**, additionally, the distributor element (20) is produced by the laser-sinter process.

10. Motor-driven hand piece according to claims 8 or 9, **characterized in that** at least the housing wall (16) is made of metal or plastic.

11. Motor-driven hand piece according to any one of claims 1 to 10, **characterized in that**, by way of the function line (36; 36A) conducting fluid, a spray nozzle arranged at the processing end (22) can be supplied with a spray fluid.

12. Motor-driven hand piece according to claim 11, **characterized in that** both the spray fluid as well as the cooling medium are formed by water and/or air, with or without additives, and are stored in a common storage reservoir (12).

13. Motor-driven hand piece according to claim 11, **characterized in that** the spray fluid and the cooling medium are stored in two separate storage reservoirs (12, 12A).

14. Motor-driven hand piece according to claim 12 or 13, **characterized in that** a revolution speed of the motor (28) can be controlled as a function of a filling level of the storage reservoir (12; 12A).

15. Motor-driven hand piece according to any one of claims 11 to 14, **characterized in that** the spray fluid and/or the cooling medium can be produced by a compressor.

16. Motor-driven hand piece according to any one of claims 1 to 15, **characterized in that** a cooling device (52) is provided, by means of which the cooling medium can be preferably cooled in a return line.

## Revendications

1. Pièce à main motorisée (4) d'un appareil de travail (2) pour le soin médical ou cosmétique des pieds, la chirurgie ou le domaine dentaire
comportant un boîtier (14), dans lequel il est prévu entre une extrémité d'assemblage (18) et une extrémité de travail (22), un logement (26) recevant un moteur électrique (28) pour l'entraînement d'un outil (30) pouvant être monté sur l'extrémité de travail (22),
et comprenant un trajet de refroidissement (34) pour guider un agent de refroidissement, et disposé au voisinage du logement (26),
**caractérisée en ce que** le trajet de refroidissement (34) est intégré dans le boîtier (14) en réalisant au moins un évidement de matériau sous la forme d'une cavité, étanche par rapport au moteur (28), dans une paroi monobloc (16) du boîtier.

2. Pièce à main motorisée selon la revendication 1, **caractérisée en ce que** le trajet de refroidissement (34) présente autour du logement (26) plusieurs sections de refroidissement.

3. Pièce à main motorisée selon la revendication 2, **caractérisée en ce que** les sections de refroidissement sont formées par des cavités formées sensiblement parallèlement, en spirale ou irrégulièrement, dont chaque fois deux cavités adjacentes sont reliées l'une à l'autre par une extrémité.

4. Pièce à main motorisée selon l'une des revendications 1 à 3, **caractérisée en ce qu**'il est en outre prévu dans la paroi (16) du boîtier un trajet fonctionnel (36 ; 36A) guidant le fluide s'étendant de l'extrémité d'assemblage (18) à l'extrémité de travail (22), et par l'intermédiaire duquel un élément fonctionnel (38), agencé sur l'extrémité de travail (22), peut être alimenté avec un fluide.

5. Pièce à main motorisée selon la revendication 4, **caractérisée en ce que** le trajet fonctionnel (36 ; 36A) guidant le fluide est intégré dans la paroi (16) du boîtier séparément du trajet de refroidissement (34).

6. Pièce à main motorisée selon la revendication 5, **caractérisée en ce que,** en plus du trajet fonctionnel (36 ; 36A) guidant le fluide, au moins un évidement fonctionnel (pour la réduction du poids ou pour recevoir des câbles ou des tuyaux) est intégré dans la paroi (16) du boîtier.

7. Pièce à main motorisée selon l'une des revendications 1 à 6, **caractérisée en ce qu**'à l'extrémité d'assemblage (18) la paroi (16) du boîtier est reliée à un élément de distribution (20) dans lequel des conduits de distribution (40a, 40b, 40c, 40d) sont insérés pour relier le trajet de refroidissement (34) et l'au moins un trajet fonctionnel (36 ; 36A) avec un conduit partiel d'alimentation respectif (42a, 42b, 42c, 42d).

8. Pièce à main motorisée selon l'une des revendications 1 à 7, **caractérisée en ce que** la paroi (16) du boîtier est fabriquée par le procédé de frittage laser.

9. Pièce à main motorisée selon la revendication 8, **caractérisée en ce que** l'élément de distribution (20) est également fabriqué par le procédé de frittage laser.

10. Pièce à main motorisée selon la revendication 8 ou 9, **caractérisée en ce qu**'au moins la paroi (16) du boîtier est fabriquée en métal ou en matière plastique.

11. Pièce à main motorisée selon l'une des revendications 1 à 10, **caractérisée en ce qu**'une buse de pulvérisation agencée à l'extrémité de travail (22) peut être alimentée avec un fluide de pulvérisation, par l'intermédiaire du conduit fonctionnel (36 ; 36A) guidant le fluide.

12. Pièce à main motorisée selon la revendication 11, **caractérisée en ce qu**'aussi bien le fluide de pulvérisation que l'agent de refroidissement sont formés par de l'eau et/ou de l'air avec ou sans additifs et sont stockés dans un réservoir commun (12).

13. Pièce à main motorisée selon la revendication 11, **caractérisée en ce que** le fluide de pulvérisation et l'agent de refroidissement sont stockés dans deux réservoirs séparés (12 ; 12A).

14. Pièce à main motorisée selon la revendication 12 ou 13, **caractérisée en ce qu**'une vitesse de rotation du moteur (28) peut être commandée en fonction d'un niveau de remplissage du réservoir (12 ; 12A) destiné à l'agent de refroidissement.

15. Pièce à main motorisée selon l'une des revendications 11 à 14, **caractérisée en ce que** le fluide de pulvérisation et/ou l'agent de refroidissement peut être produit par un compresseur (48).

16. Pièce à main motorisée selon l'une des revendications 1 à 15, **caractérisée en ce qu**'il est prévu un équipement de refroidissement (52) au moyen duquel l'agent de refroidissement peut être refroidi de préférence dans un conduit de retour.
